# EUROPÄISCHE PATENTSCHRIFT

(11) **EP 0 832 623 B1**
(45) Veröffentlichungstag und Bekanntmachung des Hinweises auf die Patenterteilung: **12.11.2003**
(21) Anmeldenummer: 97114327.6
(22) Anmeldetag: 20.08.1997
(51) Int. Cl.: A61F 5/01

(54) **Abspreizorthese**
Spreader orthosis
Orthèse d'écartement

(30) Priorität: 24.08.1996 DE 29614739 U
(43) Veröffentlichungstag der Anmeldung: 01.04.1998
(73) Patentinhaber: Frauenberger, Karl-Andreas, 30938 Burgwedel (DE); Behfar, Amir Said, Dr., 30938 Burgwedel (DE)
(72) Erfinder: Frauenberger, Karl-Andreas, 30938 Burgwedel (DE); Behfar, Amir Said, Dr., 30938 Burgwedel (DE)
(74) Vertreter: König, Norbert

(56) Entgegenhaltungen:
- DE-A- 4 438 068
- FR-A- 2 321 267
- US-A- 2 815 021
- US-A- 3 892 231

## Beschreibung

Die Erfindung betrifft eine Abspreizorthese für Spastiker.

Schwerst cerebral geschädigte Kinder entwickeln häufig eine Tetraspastik. Dabei kommt es zu erheblichen Kontrakturen der Extremitäten. Eine Adduktionskontraktion der Beine führt zu erheblichen Schwierigkeiten der Hygiene im Intimbereich und führt leichter eine Luxation der Hüftgelenke herbei.

Bisher bekannte Orthesen sind teuer und müssen in der Regel speziell auf die Größe der Patienten zugeschnitten sein. Sie sind ferner in der Anwendung umständlich.

Aus der US-A-2 815 021 ist eine Spreizorthese für Patienten nach einer Hüftgelenkoperation bekannt, durch die der Heilungsprozess und die Wiederherstellung des Gelenkes gefördert werden soll. Die Spreizorthese wird an den Füßen oder an den Unterschenkeln angelegt und besteht aus einem verlängerbaren Element nach Art einer Spannschraube an dessen beiden Enden Stützelemente für die Füße bzw. die Unterschenkel angeordnet sind.

Die Aufgabe der vorliegenden Erfindung besteht deshalb darin, eine Abspreizorthese für Spastiker zu schaffen, die einfach und preiswert herstellbar sowie einfach und sicher anlegbar und variabel ist.

Diese Aufgabe wird durch die Erfindung gemäß Anspruch 1 gelöst.

Vorteilhafte und zweckmäßige Weiterbildungen der Erfindung nach Anspruch 1 sind in den Unteransprüchen angegeben.

Die erfindungsgemäße Abspreizorthese ist einfach im Aufbau, sicher anlegbar, preiswert herstellbar und anbietbar. Sie ist modular aufgebaut, wodurch sie kompakt lagerbar und verpackbar ist. Vorzugsweise ist die Abspreizorthese verstellbar, so dass sie praktisch mit noch wachsenden Patienten, bspw. spastischen Kindern, sozusagen "mitwächst". Die Anwendung der erfindungsgemäßen Abspreizorthese schränkt nur das Zusammenführen der Knie und Beine der Patienten ein und führt zu einem Abbau von Verkrampfungen. Die Gefahr einer Luxation der Hüftgelenke wird verringert. Die Hygiene im Intimbereich wird erleichtert.

Die Erfindung soll nachfolgend anhand eines in der beigefügten Zeichnung dargestellten Ausführungsbeispieles näher erläutert werden.

Es zeigt
- Fig. 1: schematisch eine Abspreizorthese in der Draufsicht,
- Fig. 2: schematisch eine perspektivische Darstellung eines Teils der Abspreizorthese nach Fig. 1 und
- Fig. 3: schematisch ein Ausführungsbeispiel eines bei der Abspreizorthese verwendeten Stützelementes für den Knie- und/oder Fußgelenkbereich.

Gleiche Bauteile in den Figuren sind mit den gleichen Bezugszeichen versehen.

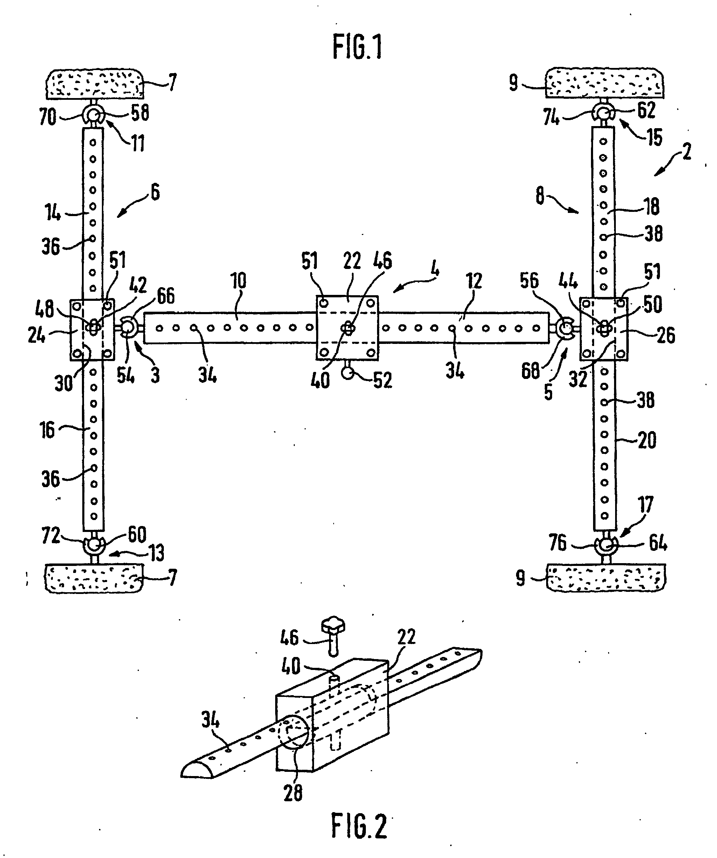

Die Fig. 1 zeigt eine Abspreizorthese 2 für Spastiker zum Anlegen zwischen den Knien und /oder Füßen von Patienten. Die Abspreizorthese 2 besteht aus einem in der Draufsicht etwa H-förmigen Gebilde mit einem in der Länge verstellbaren Mittelstück 4 und mit an dessen beiden Enden durch Gelenke 3, 5 beweglich angebrachten Querstücken 6 und 8, die ebenfalls in der Länge verstellbar sind und zur Abstützung der Knie und/oder Fußgelenke dienen.

Die Querstücke 6 und 8 weisen beidendig mittels Gelenken 11, 13 und 15, 17 beweglich angebrachte Stützelemente 7, 9, bspw. in Form von Schaum-, Gel- oder Luftpolstern, auf.

Das Mittelstück 4 und die Querstücke 6 und 8 bestehen jeweils aus zwei in ihren Längsrichtungen relativ zueinander verstellbaren Stangen 10, 12; 14, 16; 18, 20, die in Verstell- und Führungsblöcken 22, 24, 26 verschiebbar gelagert sind, welche für die Stangen Führungskanäle 28, 30, 32 aufweisen, deren Querschnitt dem Querschnitt der Stangen entspricht. Die Stangen und die Führungskanäle weisen vorzugsweise einen Querschnitt, bspw. rechteck-, trapez-, halboval-, dreieck- oder schwalbenschwanzförmigen Querschnitt auf, welcher ein Verdrehen der Stangen in den Verstell- und Führungsblöcken verhindert. Das Mittelstück und die Querstücke können auch jeweils aus zwei mit ihren flachen Seiten aufeinander liegenden Halbrundstangen bestehen, bei denen allerdings der Nachteil auftreten kann, dass durch Verdrehen im Verstell- und Führungsblock die Ausrichtung der Bohrungen der Stangen und der Verstell- und Führungsblöcke miteinander erschwert wird.

Die Stangen 10, 12, 14, 16, 18, 20 weisen miteinander ausrichtbare beabstandete Bohrungen 34, 36, 38 auf, die mit wenigstens einer Bohrung 40, 42, 44 der Verstell- und Führungsblöcke ausrichtbar sind, in die arretierbare Verstellstifte 46, 48, 50 einführbar sind zur Fixierung der jeweils eingestellten Länge des Mittelstücks und der Querstücke.

Die Verstell- und Führungsblöcke 22, 24, 26 können aus zwei mittels Schrauben 51 verschraubbaren Teilen oder auch aus einem Stück bestehen.

Die Verstell- und Führungsblöcke 22, 24, 26 und die Stützelemente 7, 9 können für die Gelenke 3, 5 und 11, 13, 15, 17 gleich ausgebildete erste Gelenkteile 52, 54, 56, 58, 60, 62, 64 und die Stangen 10, 12, 14, 16, 18, 20 des Mittelstückes 4 und der Querstücke 6, 8 endseitig an ihren freien Enden den ersten Gelenkteilen zugeordnete und mit diesen zusammenwirkende, gleich ausgebildete zweite Gelenkteile 66, 68, 70, 72, 74, 76 aufweisen.

Durch die oben beschriebene modulare Ausbildung sind die Verstell- und Führungsblöcke, die Stangen und die Polster untereinander austauschbar, und es braucht durch diese Modulbauweise nur ein Minimum an Einzelteilen hergestellt und bereitgehalten zu werden.

Das Mittelstück und die Querstücke bestehen vorzugsweise aus Kunststoff, bspw. Polykarbonat.

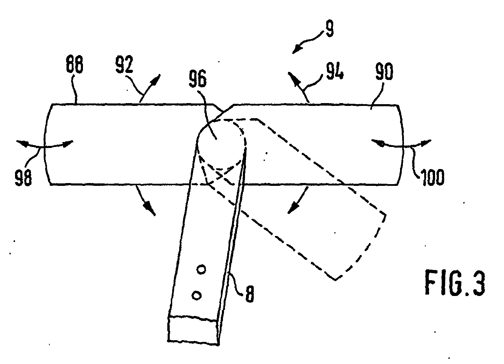

Vorzugsweise bestehen die Stützelemente 9 aus zwei schwenkbar oder gelenkig miteinander und mit dem Querstück 8 verbundenen, vorzugsweise mit Polstern versehenen Teilen 88, 90, vgl. Fig. 3, wobei vorzugsweise die beiden Teile 88, 90 je für sich in einer senkrecht zur Längsachse des Querstückes 8 verlaufenden Ebene verschwenkbar sind, vgl. Pfeile 92, 94 in Fig. 3, vorzugsweise um eine gemeinsame Schwenkachse 96. Hierdurch ist eine gerade bis beliebig winklige Lage der beiden Stützelementteile 88, 90 relativ zueinander einstellbar zur Verbesserung des Tragekomforts für die verschiedensten Liege- und Sitzpositionen eines Spastikers.

Zusätzlich kann auch noch eine gewisse Beweglichkeit der Stützelementteile 88, 90 in Richtung des Querstückes 8 vorgesehen werden zur besseren Anpassung an die jeweiligen anatomischen Gegebenheiten, vgl. Pfeile 98, 100, wobei vorgesehene Polster bereits eine gewisse Beweglichkeit in dieser Richtung zulassen.

Die Stützelemente 80, 82 sind vorzugsweise schalenförmig ausgebildet und weisen je für sich ein Mittel zur Befestigung am Ober- und Unterschenkel auf, bspw. in Form von Klettverschlussbändern (nicht dargestellt).

Die Handhabung der Abspreizorthese ist wie folgt:

Zunächst wird die Länge des Mittelstückes 4 auf den Abstand zwischen Knie und Fußgelenk eingestellt. Danach werden die Querstücke zwischen den Beinen mit ihren Stützelementen an die Innenseite des Kniebereichs und der Fußgelenkbereiche angelegt und dort fixiert, bspw. mit Hilfe von Klettverschlussbändern (nicht dargestellt). Das Maß der Spreizung der Knie und Füße kann durch Einstellung der Länge der Querstücke 6 und 8 eingestellt werden.

Durch die modulare Bauweise der Abspreizorthese ist es möglich, die Querstücke auch allein ohne das Mittelstück zu verwenden für das Auseinanderspreizen nur der Knie, insbesondere für Kleinkinder, und/oder der Füße.

## Patentansprüche

1. Abspreizorthese für Spastiker mit an den Beinen anlegbaren Stützelementen, die beweglich mit einem in der Länge verstellbaren Querstück verbunden sind, **dadurch gekennzeichnet, dass** die Stützelemente (7, 9) jeweils Stützelemente (9) für die Knie und Stützelemente (7) für die Fußgelenke beinhalten, wobei die Stützelemente (9) für die Knie aus zwei schwenkbar oder gelenkig miteinander und mit dem Querstück (8) verbundenen Stützelementteilen (88, 90) bestehen.

2. Abspreizorthese nach Anspruch 1, **dadurch gekennzeichnet, dass** zwei Querstücke (6, 8) für die Knie- und Fußgelenke vorgesehen sind, die im Wesentlichen mittig über Gelenke (3, 5) beweglich mit einem länglichen, stangenförmigen Mittelstück (4) verbunden sind.

3. Abspreizorthese nach Anspruch 1 oder 2, **dadurch gekennzeichnet, dass** das Mittelstück (4) und die Querstücke (6, 8) längenverstellbar ausgebildet sind.

4. Abspreizorthese nach Anspruch 1, 2 oder 3, **dadurch gekennzeichnet, dass** das Mittelstück (4) und die Querstücke (6, 8) aus wenigstens zwei in Längsrichtung relativ zueinander verschiebbaren Stangen (10, 12, 14, 16, 18, 20) bestehen, welche in Verstell- und Führungsblöcken (22, 24, 26), welche dem Stangenquerschnitt entsprechende Führungskanäle (28, 30, 32) aufweisen, verschiebbar und feststellbar angeordnet sind.

5. Abspreizorthese nach Anspruch 4, **dadurch gekennzeichnet, dass** die Stangen (10, 12, 14, 16, 18, 20) miteinander ausrichtbare beabstandete Bohrungen (34, 36, 38) aufweisen, die mit wenigstens einer Bohrung (40, 42, 44) der Verstellund Führungsblöcke (22, 24, 26) ausrichtbar sind, in die arretierbare Verstellstifte (46, 48, 50) einführbar sind zur Fixierung der jeweils eingestellten Länge des Mittelstückes (4) und der Querstücke (6, 8).

6. Abspreizorthese nach Anspruch 4 oder 5, **dadurch gekennzeichnet, dass** die Verstell- und Führungsblöcke (22, 24, 26) aus zwei mittels Schrauben (51) verschraubbaren Teilen oder aus einem Stück bestehen.

7. Abspreizorthese nach Anspruch 4 oder 5, **dadurch gekennzeichnet, dass** die Stangen (10, 12, 14, 16, 18, 20) Halbrundstangen sind, die mit ihren flachen Seiten aufeinander verschiebbar angeordnet sind.

8. Abspreizorthese nach Anspruch 4 oder 5, **dadurch gekennzeichnet, dass** die Stangen (10, 12, 14, 16, 18, 20) und die Führungskanäle (28, 30, 32) einen ein Verdrehen der Stangen in den Verstell- und Führungsblöcken (22, 24, 26) verhindernden Querschnitt aufweisen.

9. Abspreizorthese nach Anspruch 8, **dadurch gekennzeichnet, dass** der Querschnitt rechteckförmig, trapezförmig, halboval, dreieckförmig oder schwalbenschwanzförmig ist.

10. Abspreizorthese nach Anspruch 1, **dadurch gekennzeichnet, dass** die Stützelementteile (88, 90) jeweils in einer senkrecht zur Längsachse des Querstückes (8) verlaufenden Ebene schwenkbar ausgebildet sind.

11. Abspreizorthese nach Anspruch 10, **dadurch gekennzeichnet, dass** die Stützelementteile (88, 90) eine gemeinsame Schwenkachse (96) aufweisen.

12. Abspreizorthese nach einem der Ansprüche 1, 10 oder 11, **dadurch gekennzeichnet, dass** die Stützelementteile (88, 90) ferner vermittels Schwenklager in Richtung des Querstücks (8) verschwenkbar angeordnet sind.

13. Abspreizorthese nach einem der Ansprüche 1 oder 10 bis 12, **dadurch gekennzeichnet, dass** die Stützelemente und Stützelementteile schalenförmig ausgebildet sind.

14. Abspreizorthese nach einem der Ansprüche 1 oder 10 bis 13, **dadurch gekennzeichnet, dass** die Stützelemente und Stützelementteile Mittel zur Befestigung am Ober- und Unterschenkel im Kniebereich sowie Mittel zur Befestigung am Bein im Fußgelenkbereich aufweisen.

15. Abspreizorthese nach einem der Ansprüche 1 oder 10 bis 14, **dadurch gekennzeichnet, dass** die Stützelemente (7, 9) und Stützelementteile (8, 90) Polster aufweisen.

## Claims

1. An orthotic device for abduction for spastic persons with support elements which can be attached to the legs, the support elements displaceably connected with a longitudinally adjustable cross-piece, **characterized in that** the support elements (7, 9) each consist of support elements (9) for the knees and support elements (7) for the foot joints, wherein the support elements (9) for the knee comprise two support element parts (88, 90) which are joined pivotably or articulately with each other and with the cross-piece.

2. An orthotic device for abduction according to claim 1, **characterized in that** the two cross-pieces (6, 8) are provided for the knee and foot joints, which are joined essentially centrally by means of articulated joints (3, 5) in a displaceable manner with a long, rod-shaped central piece (4).

3. An orthotic device for abduction according to claim 1 or 2, **characterized in that** the central piece (4) and the cross-pieces (6, 8) have a longitudinally adjustable construction.

4. An orthotic device for abduction according to claim 1, 2 or 3, **characterized in that** the central piece (4) and the cross-pieces (6, 8) consist of at least two rods (10, 12, 14, 16, 18, 20) which can be displaced relative to each other in the longitudinal direction and are provided in a displaceable and fastenable manner in adjusting and guide blocks (22, 24, 26) having guide channels (28, 30, 32) corresponding to the cross-section of the rods.

5. An orthotic device for abduction according to claim 4, **characterized in that** the rods (10, 12, 14, 16, 18, 20) have spaced holes (34, 36, 38) which can be aligned with each other and can be aligned with at least one hole (40, 42, 44) of the adjusting and guide blocks (22, 24, 26) into which locking pins (46, 48, 50) can be introduced to fix the respective adjusted length of the central piece (4) and the cross-pieces (6, 8).

6. An orthotic device for abduction according to claim 4 or 5, **characterized in that** the adjusting and guide blocks (22, 24, 26) comprise two parts which can be bolted together by bolts (51) or are made from one piece.

7. An orthotic device for abduction according to claim 4 or 5, **characterized in that** the rods (10, 12, 14, 16, 18, 20) are half-round rods positioned against each other with their flat side in a displaceable manner.

8. An orthotic device for abduction according to claim 4 or 5, **characterized in that** the rods (10, 12, 14, 16, 18, 20) and the guide channels (28, 30, 32) have a cross-section which prevents the rotation of the rods in the adjusting and guide blocks (22, 24, 26).

9. An orthotic device for abduction according to claim 8, **characterized in that** the cross-section is rectangular, trapezoidal, semi-elliptic, triangular or dovetail-shaped.

10. An orthotic device for abduction according to claim 1, **characterized in that** the support elements (88, 90) each are constructed pivotably in a plane extending perpendicularly to the longitudinal axis of the cross-piece (8).

11. An orthotic device for abduction according to claim 10, **characterized in that** the support element parts (88, 90) have a common pivoting axis (96).

12. An orthotic device for abduction according to any one of the claims 1, 10 or 11, **characterized in that** the support element parts (88, 90) are pivotably provided by means of a pivoting bearing in the direction of the cross-piece (8).

13. An orthotic device for abduction according to any one of the claims 1 or 10 to 12, **characterized in that** the support elements and the support element parts have a shell-shaped construction.

14. An orthotic device according to any one of the claims 1 or 10 to 13, **characterized in that** the support elements and support element parts have means for the fastening on the upper and lower legs in the region of the knee as well as means for fastening on the leg in the region of the foot joint.

15. An orthotic device for abduction according to any one of the claims 1 or 10 to 14, **characterized in that** the support elements (7, 9) and the support element parts (88, 90) have cushions.

## Revendications

1. Orthèse d'écartement pour les handicapés moteur avec des éléments de soutien adaptables sur les jambes, éléments mobiles et reliés à une pièce transversale réglable en longueur. L'orthèse est **caractérisée par le fait que** les éléments de soutien (7, 9) comprennent des éléments de soutien (9) pour les genoux et des éléments de soutien (7) pour les chevilles, les éléments de soutien (9) pour les genoux comprennent deux parties d'éléments de soutien (88, 90) pivotables ou articulées, reliées l'une à l'autre et à une pièce transversale (8).

2. Orthèse d'écartement selon la revendication 1, **caractérisée par le fait que** deux pièces transversales (6, 8) sont prévues pour les articulations des genoux et les chevilles et reliées à une pièce médiane (4) longitudinale et en forme de cordon, mobile essentiellement au milieu au-dessus des articulations (3, 5).

3. Orthèse d'écartement selon la revendication 1 ou 2, **caractérisée par le fait que** la pièce médiane (4) et les pièces transversales (6, 8) sont constituées pour être réglées en longueur.

4. Orthèse d'écartement selon la revendication 1, 2 ou 3, **caractérisée par le fait que** la pièce médiane (4) et les pièces transversales (6, 8) comprennent au moins deux tiges mobiles relativement l'une par rapport à l'autre dans le sens de la longueur (10, 12, 14, 16, 18, 20) qui sont placées dans des blocs de réglage et de guidage (22, 24, 26) mobiles et réglables qui comportent des gouttières de guidage (28, 30, 32) correspondant à la coupe transversale des tiges.

5. Orthèse d'écartement selon la revendication 4, **caractérisée par le fait que** les tiges (10, 12, 14, 16, 18, 20) présentent des perforations (34, 36, 38) espacées orientables entre elles qui sont orientables avec au moins une perforation (40, 42, 44) des blocs de réglage et de guidage (22, 24, 26), dans lesquels il est possible d'introduire des broches de réglage fixables (46, 48, 50), destinées à fixer la longueur réglée de la pièce médiane (4) et des pièces transversales (6, 8).

6. Orthèse d'écartement selon la revendication 4 ou 5, **caractérisée par le fait que** les blocs de réglage et de guidage (22, 24, 26) sont constitués de deux parties vissables à l'aide de vis (51) ou d'une seule partie.

7. Orthèse d'écartement selon la revendication 4 ou 5, **caractérisée par le fait que** les tiges (10, 12, 14, 16, 18, 20) sont semi-circulaires, mises au point pour être déplacées l'une par rapport à l'autre sur leur côté plat.

8. Orthèse d'écartement selon la revendication 4 ou 5, **caractérisée par le fait que** les tiges (10, 12, 14, 16, 18, 20) et les gouttières de guidage (28, 30, 32) présentent une coupe transversale empêchant que les tiges se tordent dans les blocs de réglage et de guidage (22, 24, 26).

9. Orthèse d'écartement selon la revendication 8, **caractérisée par le fait que** la coupe transversale est rectangulaire, trapézoïdale, semi ovale, triangulaire ou en forme de queue d'aronde.

10. Orthèse d'écartement selon la revendication 1, **caractérisée par le fait que** les parties d'éléments de soutien (88, 90) sont montées de manière à pivoter sur un plan vertical par rapport à l'axe longitudinal de la pièce transversale (8).

11. Orthèse d'écartement selon la revendication 10, **caractérisée par le fait que** les parties d'éléments de soutien (88, 90) présentent un axe de pivotement commun (96).

12. Orthèse d'écartement selon l'une des revendications 1, 10 ou 11, **caractérisée par le fait que** les parties d'éléments de soutien (88, 90) sont mises au point pour pivoter à l'aide d'un palier de pivotement en direction de la pièce transversale (8).

13. Orthèse d'écartement selon l'une des revendications 1 ou 10 à 12, **caractérisée par le fait que** les éléments de soutien et les parties d'éléments de soutien sont en forme de cuvette.

14. Orthèse d'écartement selon l'une des revendications 1 ou 10 à 13, **caractérisée par le fait que** les éléments de soutien et les parties d'éléments de soutien présentent des possibilités de fixation sur la cuisse ou le mollet dans la zone du genou et également des possibilités de fixation sur la jambe dans la zone de la cheville.

15. Orthèse d'écartement selon l'une des revendications 1 ou 10 à 14, **caractérisée par le fait que** les éléments de soutien (7, 9) et les parties d'éléments de soutien (8, 90) présentent des rembourrages.
